(19) **Europäisches Patentamt**
**European Patent Office**
**Office européen des brevets**

(11) **EP 2 664 317 B1**

(12) **EUROPEAN PATENT SPECIFICATION**

(45) Date of publication and mention
of the grant of the patent:
**19.06.2019 Bulletin 2019/25**

(21) Application number: **12733966.1**

(22) Date of filing: **12.01.2012**

(51) Int Cl.:
*A61Q 1/02* *(2006.01)*          *A61K 8/02* *(2006.01)*
*A61K 8/25* *(2006.01)*          *A61K 8/895* *(2006.01)*
*C03B 37/00* *(2006.01)*          *C03C 3/00* *(2006.01)*
*C03C 17/30* *(2006.01)*

(86) International application number:
**PCT/JP2012/000152**

(87) International publication number:
**WO 2012/096182 (19.07.2012 Gazette 2012/29)**

(54) **GLASS FLAKE AND COSMETICS COMPRISING SAME**

GLASPLÄTTCHEN UND KOSMETIKA DAMIT

FLOCON DE VERRE ET COSMÉTIQUES COMPRENANT CELUI-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.01.2011 JP 2011006245**

(43) Date of publication of application:
**20.11.2013 Bulletin 2013/47**

(73) Proprietor: **Nippon Sheet Glass Company, Limited
Tokyo 108-6321 (JP)**

(72) Inventors:
• **TAI, Nobuaki
Tokyo 108-6321 (JP)**
• **MAEDA, Takeshi
Tokyo 108-6321 (JP)**

(74) Representative: **Hart-Davis, Jason et al
Cabinet Beau de Loménie
158, rue de l'Université
75340 Paris Cedex 07 (FR)**

(56) References cited:
**EP-A1- 1 600 145          EP-A1- 2 008 641
WO-A1-02/100356          WO-A1-2008/133042
JP-A- 6 087 720          JP-A- 2002 322 015
JP-A- 2009 161 598          US-A1- 2004 156 809
US-A1- 2010 203 097          US-A1- 2010 284 957**

Remarks:
The file contains technical information submitted after
the application was filed and not included in this
specification

**Description**

TECHNICAL FIELD

The present invention relates to glass flakes and cosmetics containing the glass flakes.

BACKGROUND ART

Natural flaky extender pigments such as talc, mica and sericite are mainly used for makeup cosmetics such as face powders, powder foundations, cheek rouges and eye shadows. In particular, mica, sericite and silica flakes (flaky silica) are widely used because they are transparent and have a soft texture, and are easy to remove when they are pressed into cakes.

However, since natural products such as mica and sericite contain impurities such as iron, they tend to darken when oil is added thereto, resulting in a problem that good color development cannot be obtained. In addition, mica and sericite have neither sufficient adhesion to the skin nor sufficient smoothness.

On the other hand, synthetic mica and amorphous flaky silica (see, for example, Patent Literature 1), and crystalline flaky silica (see, for example, Patent Literature 2) also are used instead of the natural products. However, these materials suffer from such a problem that they do not have sufficient strength and thus are crushed when they are mixed with other powder materials. The above-mentioned amorphous flaky silica and crystalline flaky silica are obtained by applying a silica coating on a substrate such as a stainless steel sheet and a steel sheet, drying it, and then peeling the coating off the substrate. Therefore, irregularities on the surface of the substrate are transferred to the flaky silica, resulting in a product with the irregularities being formed on one surface thereof (the surface that has come into contact with the substrate during production thereof). Thus, the surface reflects light, which makes it difficult to maintain the transparency of the flaky silica, and the resulting flaky silica is too glossy.

Furthermore, makeup cosmetics containing irregular-shaped silica gel are also proposed. Silica gel is a porous material. Therefore, cosmetics containing such silica gel tend to absorb moisture of the skin, resulting in a rough texture and poor spreading on the skin.

On the other hand, mica and synthetic mica are too glossy. Therefore, cosmetics containing these mica and synthetic mica also are too glossy, resulting in a problem that a natural makeup effect cannot be obtained, that is, a natural skin color can hardly be obtained.

There have also been proposed cosmetics having improved smoothness on the skin and improved adhesion to the skin by subjecting an extender pigment to surface treatment (by coating the surface of the extender pigment with a particular treatment agent). However, it is also difficult to impart sufficient smoothness to these cosmetics.

Methyl hydrogen silicone, for example, is known as a treatment agent used for surface treatment of inorganic powders used as extender pigments. Surface treatment with methyl hydrogen silicone is relatively effective in improving the smoothness on the skin, the adhesion to the skin, etc. (see, for example, Patent Literature 3). Methyl hydrogen silicone contains Si-H groups. The silicone is chemically bonded to the surface of an inorganic powder through these Si-H groups while the silicones are cross-linked and polymerized. Thus, a coating is formed on the surface of the inorganic powder. This coating imparts water repellency to the inorganic powder. Therefore, with the use of such an inorganic powder as an extender pigment, the resulting cosmetics prevent contamination with sweat and sebum on the skin surface, have a fine and smooth texture, and further have good feeling of use.

However, it is highly probable that many Si-H groups remain unreacted in the coating formed of methyl hydrogen silicone. Therefore, in the case where a cosmetic contains an inorganic powder having such a coating, hydrogen gas may be generated depending on the other components of the cosmetic. Therefore, in the case where a cosmetic contains an inorganic powder having such a coating, there is a concern about the expansion of a container for the cosmetic depending on the other components of the cosmetic (see, for example, Patent Literature 4).

CITATION LIST

Patent Literature

Patent Literature 1 JP 06(1994)-087720 A
Patent Literature 2 JP 04(1992)-145011 A
Patent Literature 3 WO 2008/133042 A1
Patent Literature 4 JP 2002-322015 A

SUMMARY OF INVENTION

Technical Problem

It is an object of the present invention to solve the above-mentioned problems and to provide glass flakes, when added to a cosmetic, capable of imparting a fine and smooth texture, excellent transparency and adhesion to the skin, and a natural finish with low gloss, that is, a so-called natural skin color, to the cosmetic, and in addition, capable of reducing the risk of expansion of a container for the cosmetic. It is another object of the present invention to provide a cosmetic containing these glass flakes.

Solution to Problem

The glass flakes of the present invention each include: a glass flake substrate; and a coating on a surface of the glass flake substrate. The glass flake substrates have an average thickness of 0.1 to 1.0 $\mu$m and an average particle size of 1 to 100 $\mu$m. The coating is formed of silicone acrylate. In the glass flakes of the present invention, a content percentage of the coatings is 0.05 to 2.20% by mass as determined by ignition loss at 625 $\pm$ 20°C.
In this description, the average thickness of the glass flake substrates refers to a numerical value obtained by, after sampling at least 100 glass flake substrates, measuring the thickness of each of the glass flake substrates with a scanning electron microscope (SEM), and then dividing the total of the thicknesses by the number of the substrates measured. In this description, the average particle size of the glass flake substrates refers to a particle size corresponding to the cumulative mass percentage of 50% (D50) in the particle size distribution measured by a laser diffraction/scattering method.
The cosmetic of the present invention contains the above-described glass flakes of the present invention.

Advantageous Effects of Invention

The glass flakes of the present invention contain the coatings formed of silicone acrylate at a mass percentage of 0.05 to 2.2 0%, as determined by ignition loss at 625 $\pm$ 20°C. As a result, a cosmetic containing the glass flakes of the present invention has a fine and smooth texture, excellent transparency, color development property and adhesion to the skin, and achieves a natural finish makeup with low gloss, that is, a makeup with a so-called natural skin color. In addition, the coatings formed of silicone acrylate do not contain groups such as Si-H groups, which may cause hydrogen gas to be generated, or even if the coatings contain such groups, the amount thereof is very small. Therefore, a cosmetic containing the glass flakes of the present invention reduces the risk of expansion of a container for the cosmetic caused by the generation of hydrogen gas.

DESCRIPTION OF EMBODIMENTS

Embodiments of the glass flakes and cosmetics of the present invention are described.
The glass flakes of the present embodiment each are provided with a coating formed of silicone acrylate on the surface of a glass flake substrate. The content percentage of the coatings in the glass flakes of the present embodiment is in the range of 0.05 to 2.20% by mass as determined by ignition loss at 625 $\pm$ 20°C. In other words, the glass flakes of the present embodiment are surface-treated with 0.05 to 2.20% by mass of silicone acrylate as determined by ignition loss. In each of the glass flakes of the present embodiment, the surface of the glass flake substrate may have a portion which is not coated with the coating.
If the coating amount of silicone acrylate (the content percentage of the coatings) in the glass flakes is less than 0.05% by mass as determined by ignition loss from the glass flakes, the water-repellent effect of the glass flakes is insufficient. Therefore, when a cosmetic containing such glass flakes is used, sweat on the skin is adsorbed onto the glass flakes, which is likely to cause dullness of the skin. On the other hand, if the coating amount is more than 2.20% by mass as determined by ignition loss from the glass flakes, the glass flakes tend to agglomerate, resulting in a problem that they are hard to disperse uniformly in the resulting cosmetic. In the present embodiment, the coating amount of silicone acrylate in the glass flakes is adjusted to fall within the above range. Thus, the cosmetic containing these glass flakes has a fine and smooth texture, excellent transparency, color development property and adhesion to the skin, and achieves a natural finish makeup with low gloss, that is, a makeup with a so-called natural skin color.
In order to further ensure that the cosmetic containing the glass flakes of the present embodiment has a fine and smooth texture, excellent transparency, color development property and adhesion to the skin, and achieves a natural finish makeup with low gloss, that is, a makeup with a so-called natural skin color, it is preferable that the coating amount of silicone acrylate in the glass flakes be within the range of 0.15 to 2.20% by mass.
In the glass flakes of the present invention, since the range of content percentages of the coatings is determined by ignition loss, the range of content percentages in which advantageous effects are achieved can be determined more precisely, compared to the case in which the content percentage of the coatings is determined by design. Thus, according to the glass flakes of the present invention, it is possible to provide a cosmetic having a fine and smooth texture, excellent transparency and adhesion to the skin, and capable of giving a natural finish with low gloss, that is, a so-called natural skin color, more reliably and stably.

The silicone acrylate used in the present embodiment is not particularly limited. However, it is desirable that the silicone acrylate further reduce the risk of generating hydrogen gas in a cosmetic containing the glass flakes of the present embodiment. Therefore, silicone acrylates not containing a Si-H group, which may cause generation of hydrogen gas, are preferably used. Examples of such silicone acrylates include (alkyl acrylate/dimethicone) copolymers, (acrylates/tridecyl acrylate/triethoxysilylpropyl methacrylate/dimethicone methacrylate) copolymers, and (acrylates/ethylhexyl acrylate/dimethicone methacrylate) copolymers. Specific examples thereof include "KP-541", "KP-574", "KP-575", "KP-543" "KP-545", "KP-549" and "KP-550" manufactured by Shin-Etsu Chemical Co., Ltd. Examples of the method for coating the glass flake substrates with silicone acrylate include a solution immersion method and a solution spray method, as exemplified in JP 61(1986)-073775 A, JP 61(1986)-017667 A, and JP 63(1988)-201041 A. For example, coatings of silicone acrylate can be formed by diluting silicone acrylate with an aromatic hydrocarbon such as toluene or xylene, an aliphatic hydrocarbon such as petroleum ether, petroleum spirit or kerosene, or isopropyl alcohol, and applying the diluted silicone acrylate to glass flake substrates to form coatings, followed by heat drying of the coatings. In this case, the amount of silicone acrylate should be adjusted appropriately so that the content percentage of the coatings to be formed is within the range of 0.05 to 2.20% by mass as determined by ignition loss, and then the silicone acrylate is applied to the glass flake substrates. Specifically, the amount of the silicone acrylate is adjusted by a method of, for example, appropriately adjusting the concentration of the silicone acrylate in a silicone acrylate solution used to form the coatings.

The glass flake substrates used in the present embodiment can be produced by, for example, a so-called blowing method as disclosed in JP 45(1970)-003541 B, or a so-called rotary method as disclosed in JP 59(1984)-021533 A and JP 02(1990)-503669 T.

In the blowing method, a nozzle is put in a liquid tank containing molten glass, air is blown through the nozzle to inflate the molten glass into a so-called balloon shape, and the balloon is drawn through rollers. Thus, glass flakes are obtained. In the rotary method, molten glass is poured continuously into a rapidly rotating flat plate or bowl, and the molten glass is stretched over the rim of the plate or the bowl. Thus, glass flakes are obtained.

Glass flake substrates having an average thickness of 0.1 to 5.0 $\mu$m and an average particle size of 1 to 1000 $\mu$m are obtained by these methods. In the present embodiment, glass flake substrates having an average thickness of 0.1 to 1.0 $\mu$m and an average particle size of 1 to 100 $\mu$m are suitably used because they can impart high transparency and adhesion to the skin as well as a natural finish with low gloss (natural skin color) to a cosmetic containing them.

Although examples of the glass flake substrates used in the present embodiment include glass compositions that are generally referred to as E-glass and C-glass, there is a concern about the elution of boron oxide resulting from the compositions thereof. Recently, there has been a growing demand for safety of cosmetics, and the "Japanese Cosmetic Ingredients Codex" edited by Examination Division, Pharmaceutical Affairs Bureau, Ministry of Health and Welfare (issued by YAKUJI NIPPO LTD. in 1997) prescribes in the category of "boron nitride" that the elution amount of boron should be 20 ppm or less. Accordingly, it is desirable that a glass used for glass flakes to be added to cosmetics should be free of boron as a component. Examples of such a glass include boron-free E-glass as disclosed in JP 2002-226732 A.

A typical composition of E glass in mass % is as follows:

$SiO_2$: 52 to 56;
$Al_2O_3$: 12 to 16;
CaO: 16 to 25;
MgO: 0 to 6;
$Na_2O + K_2O$: 0 to 2 (preferably 0 to 0.8);
$B_2O_3$: 5 to 13; and
$F_2$: 0 to 0.5.

A typical composition of boron-free E glass in mass % is as follows:

$SiO_2$: 55 to 67;
$Al_2O_3$: 5 to 15;
CaO: 17 to 25;
MgO: 0 to 6;
$Na_2O + K_2O$: 0 to 5; and

TiO$_2$: 0 to 2.

The boron-free glass that can be used is, for example, a glass composition containing, in terms of mass %:

$$60 < \text{SiO}_2 \leq 65;$$

$$8 \leq \text{Al}_2\text{O}_3 \leq 15;$$

$$47 \leq (\text{SiO}_2 - \text{Al}_2\text{O}_3) \leq 57;$$

$$1 \leq \text{MgO} \leq 5;$$

$$18 \leq \text{CaO} \leq 30;$$

$$0 < \text{Li}_2\text{O} \leq 4;$$

$$0 < (\text{Li}_2\text{O} + \text{Na}_2\text{O} + \text{K}_2\text{O}) \leq 4;$$

and

$$0 \leq \text{TiO}_2 \leq 5.$$

This glass composition is substantially free of B$_2$O$_3$, F, ZnO, BaO, SrO and ZrO$_2$. This glass composition is disclosed by the present applicant in WO 2007/148758.

An another example of the boron-free glass that can be used is a glass composition containing, in terms of mass %:

$$59 \leq \text{SiO}_2 \leq 65;$$

$$8 \leq \text{Al}_2\text{O}_3 \leq 15;$$

$$47 \leq (\text{SiO}_2 - \text{Al}_2\text{O}_3) \leq 57;$$

$$1 \leq \text{MgO} \leq 5;$$

$$20 \leq \text{CaO} \leq 30;$$

$$0 < (\text{Li}_2\text{O} + \text{Na}_2\text{O} + \text{K}_2\text{O}) < 2;$$

and

$$0 \leq \mathrm{TiO_2} \leq 5.$$

This glass composition is substantially free of B$_2$O$_3$, F, ZnO, BaO, SrO and ZrO$_2$. This glass composition is disclosed by the present applicant in WO 2006/068255.

The phrase "substantially free of a component" means that the component is not allowed to be contained except for the case where it is inevitably contained, for instance, due to impurities in industrial raw materials. Specifically, it means that the contents of B$_2$O$_3$, F, ZnO, BaO, SrO and ZrO$_2$ are each less than 0.1% by mass (preferably 0.05% by mass, and more preferably 0.03% by mass).

The glass flakes described in the present embodiment each include a glass flake substrate and a coating formed of silicone acrylate and provided on the surface of the glass flake substrate. In addition, the glass flakes may contain a surfactant; a silane coupling agent; a titania coupling agent; polymer compounds such as nylon, polymethylmethacrylate, polyethylene, fluororesin and polyamino acid; hydrogenated lecithin; acylated collagen; metallic soap; lipophilic wax; polyhydric alcohol ester, etc. in combination.

The cosmetic of the present embodiment contains the above-described glass flakes of the present embodiment.

The cosmetic of the present embodiment may contain other components which are commonly used for cosmetics appropriately as needed, in addition to the above-mentioned glass flakes. Examples of the other components include inorganic powders, organic powders, pigments, dyes, oil components, organic solvents, resins, and plasticizers. Examples of the inorganic powders include talc, kaolin, mica, sericite, other types of micas, magnesium carbonate, calcium carbonate, aluminum silicate, magnesium silicate, and clays.

Examples of the organic powders include: powders such as nylon powder, polyethylene powder, polystyrene powder, epoxy powder, and acrylic powder; and plastic beads made of nylon and acryl.

Examples of the pigments include: inorganic white pigments such as microcrystalline cellulose, titanium oxide, zinc oxide, and zirconium oxide; inorganic red pigments such as iron oxide and iron titanate; inorganic yellow pigments such as yellow iron oxide and yellow ocher; inorganic black pigments such as black iron oxide and carbon black; inorganic green pigments such as chromium oxide, chromium hydroxide, and cobalt titanate; inorganic blue pigments such as Prussian blue; pearl luster pigments such as flaky glass and silica coated with titanium oxide or iron oxide, titanium oxide coated mica (mica powder coated with titanium oxide), and bismuth oxychloride; and metallic powder pigments such as aluminum powder and copper powder.

Examples of the dyes include: organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404; organic pigments such as zirconium lakes, barium lakes, and aluminum lakes of Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1; and natural dyes such as chlorophyll and beta-carotene.

Examples of the oil components include: hydrocarbons such as squalane, liquid paraffin, vaseline, microcrystalline wax, ozokerite, ceresin, myristic acid, palmitic acid, stearic acid, oleic acid, isostearic acid, cetyl alcohol, hexadecyl alcohol, oleyl alcohol, cetyl-2-ethylhexanoate, 2-ethylhexyl palmitate, 2-octyldodecyl myristate, neopentyl glycol di-2-ethylhexanoate, glycerol tri-2-ethylhexanoate, 2-octyldodecyl oleate, isopropyl myristate, glycerol triisostearate, glycerol tri-coconut oil fatty acid, olive oil, avocado oil, yellow beeswax, myristyl myristate, mink oil, and lanoline; silicone oil; higher fatty acid; esters of oils and fats; higher alcohol; and wax.

The cosmetic may further contain: organic solvents such as acetone, toluene, butyl acetate, and acetate ester; resins such as alkyd resin and urea resin; plasticizers such as camphor and acetyl tributyl citrate; ultraviolet absorbers; antioxidants; preservatives; surfactants; moisturizers; fragrances; water; alcohol; and thickeners.

There are various forms of cosmetics. Examples thereof include powder, cake, pencil, stick, ointment, liquid, emulsion, and cream forms. Examples of these cosmetics include: facial skin care cosmetics such as skin lotions, skin emulsions, and skin creams; and makeup cosmetics such as foundations, lipsticks, eye shadows, cheek rouges, eyeliners, nail enamels, and mascaras.

EXAMPLES

Hereinafter, the present invention is described in more detail by way of examples and comparative examples, but the present invention is not limited to the following examples as long as it does not depart from the scope thereof.

(Examples 1 to 11)

Samples of the glass flakes of the present invention were prepared as Examples 1 to 11.

First, glass flake substrates were prepared by a blowing method, using E-glass and glass containing no boron (boron-free glass) each having the composition shown in Table 1. Specifically, an E-glass composition or a boron-free glass composition was put into a melting tank that had been heated to 1200°C or higher so as to melt the glass. Next, a nozzle was put into the melting tank, the molten glass was formed into a thin glass while air was blown into the tank through the nozzle, and the thin glass was continuously drawn out of the tank by rollers. A glass with an average thickness of 0.4 $\mu$m and a glass with an average thickness of 0.7 $\mu$m were obtained with different amounts of air blown into the tank and at different roller rotation speeds. After that, the glasses were each pulverized and classified. Thus, glass flake substrates with an average particle size of 10 $\mu$m and glass flake substrates with an average particle size of 25 $\mu$m were obtained. The pulverization was carried out with a jet mill type pulverizer (product name "LABO JET LJ" manufactured by Nippon Pneumatic Mfg. Co., Ltd.). The particle size was adjusted by sieve classification. The sieve classification was carried out with an electromagnetic sieve shaker (product name "RETSCH SIEVE SHAKER, type VIBRO" manufactured by RETSCH Co., Ltd.), and a sieve was selected appropriately depending on the target particle size distribution. The average particle size was determined with a laser diffraction particle size distribution analyzer (product name "Microtrack (registered trademark) HRA" manufactured by Nikkiso Co., Ltd.).

(Table 1)

|  |  | (Unit: mass%) |
| --- | --- | --- |
| Components | E-glass | Boron-free glass |
| $SiO_2$ | 54.7 | 61.1 |
| $Al_2O_3$ | 14.0 | 11.2 |
| CaO | 23.4 | 21.7 |
| MgO | 0.3 | 3.1 |
| $Na_2O$ | 0.4 | 1.3 |
| $K_2O$ | 0.2 | 0.6 |
| $Li_2O$ | - | 0.6 |
| $B_2O_3$ | 5.8 | - |
| Others | 1.2 | 0.4 |

A coating of silicone acrylate ("KP-541" manufactured by Shin-Etsu Chemical Co., Ltd.) was formed on each of the obtained glass flake substrates by the following method. The silicone acrylate "KP-541" used in these examples does not contain a Si-H group. First, "KP-541" was diluted with isopropyl alcohol. Then, the diluted "KP-541" was applied to the glass flake substrates by a solution immersion method, followed by drying of the coatings at 70°C for 4 hours and baking at 160°C for 2 hours. Thus, the coatings were formed. Samples of glass flakes of Examples 1 to 11 were prepared in the manner as described above. In these examples, the amount of the silicone acrylate contained in the glass flakes in each example was adjusted by appropriately adjusting the concentration of the silicone acrylate in a silicone acrylate solution used. Table 2 shows the type of glass used for the glass flakes, and the average thickness and average particle size of the glass flake substrates in each of Examples 1 to 11.

Next, the method for determining the ignition losses of these samples will be described. The glass flakes of each of Examples 1 to 11 thus prepared (the glass flakes each including a glass flake substrate and a coating of silicone acrylate provided on the surface of the substrate) were dried at 110 $\pm$ 5°C for at least 60 minutes, and then were ignited at 625°C for at least 15 minutes. The mass reduction (loss) of the glass flakes due to this ignition was determined, so that the percentage of the loss from the mass of the glass flakes before the ignition was calculated. Thus, the ignition loss was obtained. Table 2 shows the ignition loss of the glass flakes in each of Examples 1 to 11.

(Table 2)

|  | EX. 1 | EX. 2 | EX. 3 | EX. 4 |
| --- | --- | --- | --- | --- |
| Glass Type | Boron-free glass | Boron-free glass | Boron-free Glass | Boron-free Glass |
| Average Thickness ($\mu$m) | 0.4 | 0.4 | 0.4 | 0.4 |
| Average Particle Size ($\mu$m) | 10 | 10 | 10 | 25 |
| Ignition Loss (mass%) | 0.05 | 0.30 | 2.43 | 0.44 |

(continued)

|  | EX. 1 | EX. 2 | EX. 3 | EX. 4 |
|---|---|---|---|---|
| Glass Type | Boron-free glass | Boron-free glass | Boron-free Glass | Boron-free Glass |
| Material of Coatings | Silicone Acrylate | | | |
|  | EX. 5 | EX. 6 | EX. 7 | EX. 8 |
| Glass Type | Boron-free Glass | Boron-free Glass | E-Glass | E-Glass |
| Average Thickness ($\mu$m) | 0.7 | 0.7 | 0.4 | 0.7 |
| Average Particle Size ($\mu$m) | 10 | 25 | 10 | 10 |
| Ignition Loss (mass%) | 0.54 | 0.15 | 0.43 | 0.25 |
| Material of Coatings | Silicone Acrylate | | | |
|  | EX. 9 | EX. 10 | EX. 11 | |
| Glass Type | Boron-free Glass | Boron-free Glass | Boron-free Glass | |
| Average Thickness ($\mu$m) | 0.4 | 0.4 | 0.4 | |
| Average Particle Size ($\mu$m) | 10 | 10 | 80 | |
| Ignition Loss (mass%) | 0.15 | 2.20 | 0.35 | |
| Material of Coatings | Silicone Acrylate | | | |

(Comparative Examples 1 to 8)

The following glass flakes were prepared as comparative examples using glass flake substrates that had been prepared in the same manner as in Examples 1 to 11. Specifically, glass flakes in which the glass flake substrates were not coated with silicone acrylate (Comparative Examples 1 and 2), glass flakes in which the glass flake substrates were coated with less than 0.05% by mass of silicone acrylate as determined by ignition loss (Comparative Examples 3 and 5), and glass flakes in which the glass flake substrates were coated with more than 2.50% by mass of silicone acrylate as determined by ignition loss (Comparative Examples 4 and 6) were prepared. In addition, the following glass flakes were prepared as comparative examples. Specifically, glass flakes in which the glass flake substrates were coated with methyl hydrogen silicone instead of silicone acrylate (Comparative Examples 7 and 8) were prepared in the same manner as in Examples 1 to 11.

Table 3 shows the type (glass type) of the substrates used in each of Comparative Examples 1 to 8, the average thickness and average particle size thereof, the ignition loss that was determined in the same manner as in Examples 1 to 11, and the material of the coatings.

(Table 3)

|  | C.EX. 1 | C.EX. 2 | C.EX. 3 | C.EX. 4 |
|---|---|---|---|---|
| Glass Type | Boron-free Glass | E-Glass | Boron-free Glass | Boron-free Glass |
| Average Thickness ($\mu$m) | 0.4 | 0.4 | 0.4 | 0.4 |
| Average Particle Size ($\mu$m) | 10 | 10 | 10 | 10 |
| Ignition Loss (mass%) | - | - | 0.03 | 2.55 |
| Material of Coatings | No Coating | | Silicone Acrylate | |
|  | C.EX. 5 | C.EX. 6 | C.EX. 7 | C.EX. 8 |
| Glass Type | Boron-free Glass | E-Glass | Boron-free Glass | E-Glass |
| Average Thickness ($\mu$m) | 0.4 | 0.4 | 0.4 | 0.4 |
| Average Particle Size ($\mu$m) | 25 | 10 | 10 | 10 |
| Ignition Loss (mass%) | 0.04 | 2.62 | 0.46 | 0.32 |
| Material of Coatings | Silicone Acrylate | | Methyl Hydrogen Silicone | |

(Examples 12 to 22 and Comparative Examples 9 to 16)

Powder foundations containing components shown in Table 4 were prepared, using the glass flakes of Examples 1 to 11 and Comparative Examples 1 to 8, and evaluated for their performance as cosmetics (Examples 12 to 22 and Comparative Examples 9 to 16).

The components (1) to (8) shown in Table 4 were mixed in a Henschel mixer to obtain a mixture. The components (9) to (13) that had been heated, dissolved and mixed were added to this mixture, and then the resulting mixture was pulverized by a pulverizer. The resulting powder was molded into a plate with a diameter of 6 cm at a pressure of 1.5 kg/cm$^2$. Thus, the powder foundations were obtained.

(Table 4)

|  | | Components | Part(s) by Mass |
|---|---|---|---|
| (1) | | Glass Flakes of EX. 1 to 11 or Glass Flakes of C. EX. 1 to 8 | 35 parts by mass |
| (2) | | Talc | 20 parts by mass |
| (3) | | Glass Flakes Coated with Titanium | 20 parts by mass |
| (4) | | Titanium dioxide | 10 parts by mass |
| (5) | | Spherical Polyethylene Powder | 5 parts by mass |
| (6) | | Colcothar | 1.0 part by mass |
| (7) | | Yellow Iron Oxide | 3.0 parts by mass |
| (8) | | Black Iron Oxide | 0.1 part by mass |
| (9) | | Silicone Oil | 1 part by mass |
| (10) | | 2-Ethylhexyl Palmitate | 9 parts by mass |
| (11) | | Sorbitan Sesquioleate | 1 part by mass |
| (12) | | Preservative | 0.3 part by mass |
| (13) | | Fragrance | 0.1 part by mass |

For Examples 12 to 22 and Comparative Examples 9 to 16 in which foundations were prepared using the glass flakes of Examples 1 to 11 and Comparative Examples 1 to 8, sensory tests were performed.

For the sensory tests, 10 panelists were selected. The average of the scores of the 10 panelists was used to evaluate the feeling of use of the cosmetics. Table 5 shows the evaluation criteria, and Table 6-1 and Table 6-2 show the evaluation results.

(Table 5)

| Evaluation Score | Feeling of Finish | Feeling of Transparency | Feeling of Use (Spreading) |
|---|---|---|---|
| 1 | Very Unnatural | Very Poor | Very Poor |
| 2 | Unnatural | Poor | Poor |
| 3 | Average | Average | Average |
| 4 | Natural | Good | Good |
| 5 | Very Natural | Very Good | Very Good |

(Table 6-1)

|  | EX. 12 | EX. 13 | EX. 14 | EX. 15 |
|---|---|---|---|---|
| Substrate Used | EX. 1 | EX. 2 | EX. 3 | EX. 4 |
| Elution Amount of Boron (ppm) | n.d.(*1) | n.d.(*1) | n.d.(*1) | n.d.(*1) |
| IR area ratio (Si-H) | 0 | 0 | 0 | 0 |

(continued)

|  | EX. 12 | EX. 13 | EX. 14 | EX. 15 |
|---|---|---|---|---|
| Substrate Used | EX. 1 | EX. 2 | EX. 3 | EX. 4 |
| Feeling of Finish | 4.5 | 5.0 | 5.0 | 5.0 |
| Feeling of Transparency | 5.0 | 5.0 | 4.5 | 5.0 |
| Feeling of Use (Spreading) | 4.5 | 5.0 | 5.0 | 5.0 |
|  | EX. 16 | EX. 17 | EX. 18 | EX. 19 |
| Substrate Used | EX. 5 | EX. 6 | EX. 7 | EX. 8 |
| Elution Amount of Boron (ppm) | n.d.(*1) | n.d.(*1) | 1 | 1 |
| IR area ratio (Si-H) | 0 | 0 | 0 | 0 |
| Feeling of Finish | 5.0 | 4.5 | 5.0 | 5.0 |
| Feeling of Transparency | 5.0 | 5.0 | 5.0 | 5.0 |
| Feeling of Use (Spreading) | 5.0 | 4.5 | 5.0 | 5.0 |
|  | EX. 20 | EX. 21 | EX. 22 |  |
| Substrate Used | EX. 9 | EX. 10 | EX. 11 |  |
| Elution Amount of Boron (ppm) | n.d.(*1) | n.d.(*1) | n.d.(*1) |  |
| IR area ratio (Si-H) | 0 | 0 | 0 |  |
| Feeling of Finish | 4.5 | 5.0 | 4.0 |  |
| Feeling of Transparency | 5.0 | 5.0 | 5.0 |  |
| Feeling of Use (Spreading) | 5.0 | 5.0 | 4.0 |  |
| (*1) n.d.: below 0.25 ppm which is the minimum limit of quantification | | | | |

(Table 6-2)

|  | C.EX. 9 | C.EX. 10 | C.EX. 11 | C.EX. 12 |
|---|---|---|---|---|
| Substrate Used | C.EX. 1 | C.EX. 2 | C.EX. 3 | C.EX. 4 |
| Elution Amount of Boron (ppm) | n.d.(*1) | 52 | n.d.(*1) | n.d.(*1) |
| IR area ratio (Si-H) | 0 | 0 | 0 | 0 |
| Feeling of Finish | 3.0 | 3.0 | 3.5 | 4.5 |
| Feeling of Transparency | 4.5 | 4.5 | 4.5 | 3.0 |
| Feeling of Use (Spreading) | 3.5 | 3.5 | 3.5 | 4.0 |
|  | C.EX. 13 | C.EX. 14 | C.EX. 15 | C.EX. 16 |
| Substrate Used | C.EX. 5 | C.EX. 6 | C.EX. 7 | C.EX. 8 |
| Elution Amount of Boron (ppm) | n.d.(*1) | n.d.(*1) | n.d.(*1) | 1 |
| IR area ratio (Si-H) | 0 | 0 | 2.3 | 2.1 |
| Feeling of Finish | 4.0 | 4.5 | 5.0 | 5.0 |
| Feeling of Transparency | 4.5 | 3.0 | 5.0 | 5.0 |
| Feeling of Use (Spreading) | 3.0 | 4.0 | 5.0 | 5.0 |
| (*1) n.d.: below 0.25 ppm which is the minimum limit of quantification | | | | |

As shown in Table 6-1, the powder foundations (Examples 12 to 22) using the glass flakes of the present invention (Examples 1 to 11) achieved evaluation scores of 4.0 or higher in all the evaluation items, and thus obtained better results than the powder foundations (Comparative Examples 9 to 16) using the glass flakes of Comparative Examples 1 to 8 shown in Table 6-2.

Since the glass flakes of Comparative Examples 1 and 2 having no coating of silicone acrylate themselves were not sufficiently smooth, the spreading on the skin and the feeling of finish of the resulting foundations (Comparative Examples 9 and 10) were both poor. Since the glass flakes of Comparative Examples 3 and 5 having a small amount of coating of silicone acrylate themselves did not have sufficient water repellency, the spreading and the feeling of finish of the resulting foundations (Comparative Examples 11 and 13) were poor. Since the glass flakes of Comparative Examples 4 and 6 having a large amount of coating of silicone acrylate did not disperse sufficiently in foundations, not only the darkening of the resulting foundations (Comparative Examples 12 and 14) tended to occur but also the feeling of use thereof was poor.

In the glass flakes having coatings formed of methyl hydrogen silicone (Comparative Examples 7 and 8), Si-H groups which may cause the generation of hydrogen gas were detected. In contrast, no Si-H group was detected in the glass flakes having coatings formed of silicone acrylate (Examples 1 to 11). Therefore, the glass flakes that satisfy the constituent features of the present invention do not cause the problem of the product stability (expansion of a container) due to the generation of hydrogen gas. The amount of Si-H groups were determined by infrared spectroscopy using a coating method.

5 g of glass flakes was put into a beaker, and n-hexane was added thereto. The glass flakes were immersed in n-hexane for 67 hours to extract the components of the glass flakes. The extract was filtered to remove insoluble components, and soluble components were dried. Thus, coating samples were prepared. The ratio of the area under the 2160 $cm^{-1}$ peak derived from Si-H groups to the area under the 2964 $cm^{-1}$ peak derived from factors other than Si-H groups was obtained from the infrared absorption spectrum of each coating sample, and defined as an IR area ratio (Si-H). Relative comparison of these IR area ratios as measures of the amount of Si-H groups was performed for evaluation.

Compared to Comparative Examples in which E-glass was used for glass flake substrates, the elution amount of boron from the glass flakes in Examples was very small, which indicated that the glass flakes in Examples were highly safe. A test solution was prepared in accordance with the method of "boron nitride" purity test (4), the elution amount of boron, in the Japanese Cosmetic Ingredients Codex, and then the solution was subjected to quantitative analysis by the atomic absorption method to determine the elution amount of boron. The method for preparing the test solution is as follows.

2.5 g of glass flakes was put into a beaker made of fluororesin, and then 10 mL of ethanol was added thereto and stirred well. Then, 40 mL of water was further added thereto and stirred well. The resulting solution was placed on a watch glass made of fluororesin, and heated on a hot plate for 1 hour. After cooling, the solution was filtered, and the residues were washed with a small amount of water. Thus, the wash liquid was mixed with the filtrate. This mixed liquid further was filtered through a membrane filter (0.22 $\mu$m). All the filtrate was poured into a beaker made of fluororesin, and 1 mL of sulfuric acid was added thereto. Then, the resulting solution was boiled on a hot plate for 10 minutes. After cooling, the solution thus obtained was poured into a volumetric flask made of polyethylene. The fluororesin beaker was washed with a small amount of water, and the wash liquid was mixed with the solution in the polyethylene volumetric flask. Thereafter, water was added thereto until exactly 50 mL of solution was obtained. Thus, a test solution was prepared.

INDUSTRIAL APPLICABILITY

A cosmetic containing the glass flakes of the present invention can be used as a cosmetic having a fine and smooth texture, excellent transparency and adhesion to the skin, and achieving a natural finish with low gloss, that is, a so-called natural skin color, and capable of reducing the risk of expansion of a container for the cosmetic caused by generation of hydrogen gas.

**Claims**

1. Glass flakes each comprising:

    a glass flake substrate; and
    a coating on the surface of the glass flake substrate, wherein
    the glass flake substrates have an average thickness of 0.1 to 1.0 $\mu$m and an average particle size of 1 to 100 $\mu$m,
    the coating is formed by applying silicone acrylate to the surface of the glass flake substrate, and
    a content percentage of the coating is 0.05 to 2.20 % by mass as determined by ignition loss at 625 $\pm$ 20°C.

**2.** The glass flakes according to claim 1, wherein the silicone acrylate does not contain a Si-H group.

**3.** The glass flakes according to claim 1, wherein the glass flake substrate is free of boron as a component.

**4.** A cosmetic comprising the glass flakes according to claim 1.


**Patentansprüche**

**1.** Glasplättchen, jeweils umfassend:

ein Glasplättchensubstrat und
eine Beschichtung auf der Oberfläche des Glasplättchensubstrats, wobei
die Glasplättchensubstrate eine durchschnittliche Dicke von 0,1 bis 1,0 $\mu$m und eine durchschnittliche Teilchengröße von 1 bis 100 $\mu$m aufweisen,
die Beschichtung durch Aufbringen von Silikonacrylat auf die Oberfläche des Glasplättchensubstrats gebildet ist,
ein durch den Glühverlust bei 625 $\pm$ 20 °C bestimmter prozentualer Beschichtungsgehalt 0,05 bis 2,20 Masse% beträgt.

**2.** Glasplättchen nach Anspruch 1, wobei das Silikonacrylat keine Si-H-Gruppe enthält.

**3.** Glasplättchen nach Anspruch 1, wobei das Glasplättchensubstrat frei von Bor als Bestandteil ist.

**4.** Kosmetikum, umfassend die Glasplättchen nach Anspruch 1.


**Revendications**

**1.** Flocons de verre comprenant chacun :

un substrat de flocon de verre ; et
un revêtement sur la surface du substrat de flocon de verre, dans lesquels
les substrats de flocons de verre présentent une épaisseur moyenne de 0,1 à 1,0 $\mu$m et une taille moyenne de particule de 1 à 100 $\mu$m,
le revêtement est formé par application d'acrylate de silicone sur la surface du substrat de flocon de verre, et
un pourcentage de teneur du revêtement est de 0,05 à 2,20 % en masse comme déterminé par perte d'allumage à 625 $\pm$ 20°C.

**2.** Flocons de verre selon la revendication 1, dans lesquels l'acrylate de silicone ne contient pas un groupe Si-H.

**3.** Flocons de verre selon la revendication 1, dans lesquels le substrat de flocon de verre est exempt de bore comme un constituant.

**4.** Cosmétique comprenant les flocons de verre selon la revendication 1.

## REFERENCES CITED IN THE DESCRIPTION

**Patent documents cited in the description**

- JP 6087720 A
- JP 4145011 A
- WO 2008133042 A1
- JP 2002322015 A
- JP 61073775 A
- JP 61017667 A
- JP 63201041 A

- JP 451970003541 B
- JP 59021533 A
- JP 2503669 T
- JP 2002226732 A
- WO 2007148758 A
- WO 2006068255 A

**Non-patent literature cited in the description**

- Japanese Cosmetic Ingredients Codex. YAKUJI NIP-PO LTD, 1997